# EUROPEAN PATENT APPLICATION

(11) **EP 2 301 333 A1**
(43) Date of publication of application: **30.03.2011**
(21) Application number: 10178450.2
(22) Date of filing: 22.07.2003
(51) Int. Cl.: A01N 1/02

(54) **Non-human sperm cell process system**

(30) Priority: 22.07.2002 US 400486 P
(62) Divisional of application: 03765919.0
(71) Applicant: XY, LLC, Navasota, TX 77868 (US)
(72) Inventor: Lindsey, Allison, C., Fort Collins, CO 80524 (US); Schenk, John, L., Fort Collins, CO 80524 (US)
(74) Representative: Jacob, Reuben Ellis

(57) **Abstract**

A semen or sperm cell process system to maintain or enhance the biological, chemical, physical, physiological, or functional attributes of sperm cells within the context of various collecting, handling, storage, transportation, separation, or insemination procedures.

## Description

### I. TECHNICAL FIELD

A semen or sperm cell process system to maintain or enhance the biological, chemical, physical, physiological, or functional attributes of sperm cells within the context of various collecting, handling, storage, transportation, separation, or insernination procedures.

### II. BACKGROUND

Effective preselection of sex has been accomplished in many species of livestock following the development of safe and reliable methods of separating sperm cells into enriched X chromosome bearing and Y chromosome bearing populations. Separation of X chromosome bearing sperm cells from Y chromosome bearing sperm cells can be accomplished as disclosed herein and as disclosed by various international patent applications, for example: PCTUS99/17165; PCT/US98/2790; PCT/US01/45237; PCT/01/18879; PCT/US01/15150; and PCT/US01/02304 and United States patent applications 091582,809; and 49/015,454, each hereby incorporated by reference. These examples of separating X chromosome bearing sperm cells from Y chromosome bearing sperm cells are not meant to limit the instant sperm cell process system invention to sperm processing technology with flow cytometry sorting devices or flow-sorting methods but rather are meant to be illustrative of various processes by which sperm cells may be separated from one another and to be illustrative of the manner in which sperm cells are collected, handled, separated, transported, used, or stored as a context in which the instant invention can be understood.

A significant problem with sperm cells that have been separated into subpopulations based upon a specific sperm cell characteristic can be lower motility, lower viability or lower fertility. One aspect of this problem may be that conventional procedures of handling and transporting semen prior to separation of sperm cells into subpopulations is typically carried out at about 5°C, or at a temperature at which sperm cell membrane lipids have transitioned from a liquid phase to a gel phase. The transition of sperm cell membranes lipids from the liquid phase to the gel phase can alter the chemical, physical, physiological, or functional attributes of sperm cells which can result in lower fertility of such sperm cells or can result in sperm cell death. Another aspect of this problem can be that the temperature at which sperm cell membrane lipids transition from the liquid phase to the gel phase can vary depending on the species of mammal from which the semen is obtained. As such, the temperature at which sperm cells for a particular species of mammal must be maintained to prevent transition of sperm cell membrane lipids from the liquid phase to the gel phase must be determined in advance of subsequent collection, handling, transportation and separation procedures. Another aspect of this problem is the conventional understanding that collected semen should be reduced in temperature to maintain sperm cell viability and fertility during subsequent handling, transportation, or separation procedures which teaches away from the present invention of incubating or maintaining the temperature of collected semen at a temperature which is substantially higher than existing conventional practice.

Another significant problem with conventional procedures for separation of sperm cells into subpopulations can that stain concentration to which sperm cells are exposed may be too high for sperm cells obtained from certain species of mammals. For example, equine sperm cells exposed to concentration of Hoechst 33342 stain conventionally used to stain bovine sperm cells may exhibit substantial loss of fertility.

Another significant problem with conventional procedures for separation of sperm cells into subpopulations can be the duration of time sperm cells are incubated in stain, such as Hoechst 33342, may be too long for certain species of mammals. For example equine sperm cells incubated in Hoechst 33342 for one hour may exhibit substantial loss of motility or fertility.

Yet another significant problem with conventional procedures for handling semen can be extension of the semen with compositions in which sperm cells exhibit substantial loss of motility or fertility.

Even though the various devices and methods of separating sperm, cells have improved significant problems remain with respect to maintaining sperm viability during collection, handling, transportation, separation, use, or storage processes.

### III. DISCLOSURE OF INVENTION

Accordingly, the broad object of the invention can be to provide devices or methods for the collection, handling, shipment, storage, or separation of semen or sperm cells to maintain sperm viability.

Another broad object of the invention can be to provide devices or methods for collecting, handling, shipment, storing, or separating semen or sperm cells obtained from various species of mammals, including, but not limited to equids, bovids, felids, ovids, canids, buffalo, oxen, elk, or swine; or obtained from prise, endangered, or rare individuals of a mammal species; or obtained from zoological specimens to maintain or enhance sperm viability.

Another significant object of the invention can be to provide devices or methods for handling and transporting sperm cells obtained from equine mammals.

Another significant object of the invention can be to provide devices or methods of separating sperm cells that can maintain greater viability of mamalian sperm cells throughout a flow-sorting process.

Another significant object of the invention can be to provide devices or methods of maintaining sperm cells at greater viability for purposes of artificial insemination of various species of mammals, such as those described above, or even artificial insemination with a low or reduced number of sperm cells compared to the usual number or typical number of sperm cells used in such artificial insemination procedures whether or not such sperm cells are separated into enriched X chromosome bearing or Y chromosome bearing sperm cells.

Another significant object of the invention can be to provide methods in which sperm cells are incubated at temperatures above which sperm cell membrane lipids transition from a liquid phase to a gel phase.

Another significant object of the invention can be to provide devices or methods for the shipment of stallion sperm prior to separation of flow-sorting of sperm cells.

### IV. BRIEF DESCRIPTION OF DRAWINGS

Figure 1 provides a graph showing that as stain concentration increases the total and progressive motility of sperm cells decreases, % dead sperm cells increases, and the ability of flow cytometry techniques to resolve X-chromosome bearing sperm cells from Y-chromosome sperm cells increases.
Figure 2 provides a graph showing that sperm cells extended in KMT remain more motile with respect to both fresh sperm samples and sperm samples stored for a duration of time at room temperature, such as 18 hours at room temperature
Figure 3 provides a graph showing that staining sperm cells at higher pH can decrease the % dead sperm cells in stained sperm cells samples and increase resolution as evaluated by flow cytometry analysis.
Figure 4 provides a graph showing that decreasing the stain incubation period from a conventional period of 60 minutes to about a 30 minute incubation period can increase motility, decrease % dead sperm cells in stained sperm cell samples, and increase resolution of X-chromosome bearing sperm cells from Y-chromosome bearing sperm cells during flow sorting of stained sperm cells.
Figure 5 provides a graph showing that the addition of a stimulant, such as caffeine, can increase motility in sperm cells.
Figure 6 provides a graph showing that total motility and progressive mobility of sperm cells with respect to using modified KMT prepared using NaH₂PO₄.
Figure 7 provides a graph showing that total motility and progressive motility of sperm cells can be increased using modified KMT prepared using NaH₂PO₄ whether or not the sperm cells are exposed to stimulant, such as caffeine.
Figure 8 provides a graph showing that temperature can be adjusted for storing, handling, transferring, or transportation of sperm cells obtained from a male of a species of mammal to increase total and progressive inotility.
Figure 9 provides a graph showing that the temperature at which sperm cells are transferred, stored, or handled prior to a staining protocol can be adjusted to increase total or progressive motility of sperm cells, or stimulated sperm cells, or sperm cells stimulated with caffeine.
Figure 10 provides a graph showing that temperature at which sperm cells are transferred, stored, or handled prior to a staining protocol can be adjusted to increase total or progressive motility of sperm cells, or stimulated sperm cells, or sperm cells stimulated with caffeine subsequent to a staining protocol.
Figure 11 provides a graph showing that % dead sperm cells in stained sperm cell samples can be reduced by storing or transporting sperm cells at 15°C.
Figure 12 provides a graph showing that sperm cells can remain more viable when sperm cell concentration during staining is at about 100 M/mL versus 200M/mL without loss of flow cytometry resolution.
Figure 13 provides a graph showing that as stain concentration increases fewer sperm cells survive and resolution increases.
Figure 14 provides a graph showing that stain time can be substantially decreased without loss of resolution between X-chromosome bearing populations and Y-chromosome bearing populations of sperm cells evaluated by flow cytometery.

### V. MODE(S) FOR CARRYING OUT THE INVENTION

A semen or sperm cell process system to maintain or enhance the biological, chemical, physical, physiological, or functional attributes of sperm cells within the context of various collecting, handling, storage, transportation, separation, or insemination procedures.

### EXAMPLE 1.

Semen was collected from three stallions of acceptable fertility, extended to about 25 x 10⁶ sperm/mL in a Tyrode's-based skim milk-glucose extender, and stored for 18 h at either about 5°C or about 15°C. Following storage, spermatozoa were centrifuged to remove seminal plasma and concentrate sperm, stained with Hoechst 33342 (Hoechst), and sorted into enriched X-chromosome bearing and Y-chromosome-bearing populations based on DNA content using an SX MoFlo® sperm sorter.

A final dose of about 20 x 10⁶ flow-sorted sperm in a volume of 300 µL was used for all insemination. Estrus was synchronized in 35 mares ages 2 to 12. Human chorionic gonadotropin (hCG; 3000 IU, iv; Chorulon®, Intervet, Millsboro, DE, USA) was administered when a dominant follicle ≥35 mm in diameter was present and mares were inseminated at approximately 30 h post-hCG. At the time of insemination, mares were assigned to 1 of 3 treatment groups: 1) sperm that had been stored at 15°C and inseminated using the videoendoscopic technique; 2) sperm stored at 5°C and also inseminated using the videoendoscopic method; and 3) sperm stored at 5°C and inseminated using the rectally guided technique.

Mares were sedated immediately prior to insemination using butorphanol (4 mg, iv; Torbugesic®, Ft. Dodge Co., Fort Dodge, IA, USA) and detomidine (6 mg, iv; Dormosedan®, Pfizer, Lees Summit, MO, USA). Mares were evaluated daily for ovulation, and only those mares ovulating within 48 h after insemination were included in the results. Pregnancy was determined ultrasonographically at 12 to 14 days post-ovulation. Mares were administered prostaglandin F₂α at day 16 post-ovulation for use in 2 subsequent cycles.

Pregnancy rates between mares inseminated heteroscopically with sorted sperm stored at 15°C was about 72% or at 5°C was about 55% as shown by Table 1. There was a tendency (P=0.12) for fewer mares to become pregnant following rectally guided insemination (38%) compared to hysteroscopic insemination (55%) when stored, sorted sperm were inseminated.

**Table 1. Pregnancy rates from hysteroscopic or rectally guided insemination of flow-sorted sperm stored at 5°C or 15°C.**

| Storage temp. (C°) | Insemination method | Mares inseminated | Mares pregnant (14 d) | Pregnancy rate |
|---|---|---|---|---|
| 15 | Hysteroscopic | 25 | 18 | 72%^{a} |
| 5 | Hysteroscopic | 22 | 12 | 55%^{a,b} |
| 5 | Rectally guided | 24 | 9 | 38%^{b} |

| | | | | |
|---|---|---|---|---|
| ^{a.b}Values in the same column with different superscripts are significantly different (P<0.05). | | | | |

A greater number of mares became pregnant following insemination with sorted sperm stored prior to sorting at 15°C as compared to 5°C. This effect was consistent across stallions as was fertility after storage at 15°C for 18 h prior to sorting, as compared to 5°C.

The expected pregnancy rate following insemination of 1 x 10⁹ stallion sperm shipped by standard methods (5°C, 12 to 72 h) is 65%. The pregnancy rate obtained in the present study (72%) is impressive, and shows dramatic improvement over that obtained (35%) with 18 h stored, flow-sorted sperm using conventional technology. This increase in fertility may reflect sperm processing prior to flow cytometry. Pregnancy rates in the present study would have been even higher except that for two days during this application of the invention pregnancy rates for all mares inseminated were extremely low.

Hysteroscopic insemination resulted in higher pregnancy rates than deep-uterine insemination. This is in contrast to Rigby et al. who reported similar pregnancy rates between videoendoseopic and rectally guided insemination when shipped sperm were used. However, those results did show a 12-percentage point advantage for hysteroscopic insemination. In contrast to that study, the present trial utilized flow-sorted sperm, which are known to be in a pro-capacitated state. In summary, excellent pregnancy rates were obtained with hysteroscopic insemination of 18 h stored, flow-sorted spermatozoa.

Pregnancy rates were higher for all three stallions when sperm was maintained at about 15°C as compared to 5°C. Hysteroscopic insemination with sperm stored at 15°C resulted in higher pregnancy rates than rectally guided insemination of sperm stored at 5°C.

As such, the sperm cell process system invention can involve obtaining sperm cells from a male of a species of mammal, maintaining the sperm cells obtained from the male species of mammal prior to artificial insemination of a female of the species of mammal at a temperature(s) selected within the range of between 5°C and 25°C that generates a higher pregnancy rate of such females of such species of mammal. With respect to equine species, and particularly with respect to the species of equids disclosed, the temperature at which sperm celts obtained from equine males are maintained in accordance with the invention to increase pregnancy rate can be between about 10°C to about 20°C, and can specifically be about 15°C. See Examples below for application involving equids. See also, Example 8 below showing an application of the invention in which sperm cells obtained from a male of a species of elk are maintained at about 20°C prior to insemination of cow elk.

The sperm cell process system invention can further include transportation of the sperm cells obtained from the male of a species of mammal maintained at temperature(s) in accordance with the invention. Such transportation may have a limited duration of less than an hour or may have a more extended duration between about 1 hour and about 72 hours, or as described may be a duration of about 18 hours.

The invention can further include the step of staining the sperm cells obtained from a male of a species of mammal as above-described which have been maintained at a temperature that generates the highest pregnancy rate of such females of such species of mammal.

The invention can further include hysteroscopic or rectally guided artificial insemination of the female of the species of mammal. Specifically, as described by the incorporation by reference included herein, hysteroscopic insemination of equine mammals with sperm cells sorted for sex preselection that may be handled in accordance with the instant invention and may further include a low number of sperm cells compared to the number of sperm cells typically used to inseminate a female of a particular species of mammal, including but not limited to equine mammals.

### EXAMPLE 2.

Semen from eight stallions was extended to about 25 x 10⁶ sperm/ml in each of four shipping extenders as set out in Table 2. During simulated shipping for 18 h, samples were held at ambient temperature (20-24°C), except those extended in INRA96, which were stored at 15°C. Following storage, samples were centrifuged at 600 x g for 10 min and pellets extended to 400 x 10⁶ sperm/ml. After incubation at 19-24°C for 1 h and dilution to 200 x 10⁶ sperm/ml, sperm were stained at 34°C with 224 µM Hoechst 33342 for 1 h, and then diluted to 100 x 10⁶ sperm/ml in KMT. To simulate sorting conditions, sperm were diluted to 700,000 sperm/ml in HBGM-3 without BSA and held at ambient temperature for 1.5 h prior to centrifugation at 850 x g for 20 main. Motility was evaluated at four chemical environments as shown by Table 2.

**Table 2. Percentage of motile sperm in samples stored in four different shipping media**

| Media | Post- ship | Pre-Stain | Post-Stain | Post-Dil. |
|---|---|---|---|---|
| EZ Mixin CST; Anim Reprod Systems, Chino, CA | 59 | 48^{a,b} | 31^{b} | 47^{a} |
| Next Generation; Exodus Breeders, York, PA | 54 | 40^{b} | 27^{b} | 36^{b} |
| KMT; J Anim Sci 1991;69:3308-3313 | 64 | 58^{a} | 46^{a} | 50^{a} |
| INRA96; IMV Technologies, France | 63 | 53^{a} | 43^{a} | 54^{a} |

| | | | | |
|---|---|---|---|---|
| ^{a,b} Values in the same column without common superscripts differ (P<0.05), Tukey's Test. | | | | |

As can be understood from the data set out by Table 2, KMT and INRA96 maintained higher motility throughout certain sperm cell process procedures.

The sperm cell process system invention can further include the step of extending sperm cells obtained from a male of a species of mammal in KMT, and specifically with respect to sperm cells obtained from the male of an equine species of mammal KMT can significantly increase sperm cell motility.

The sperm cell process system invention can further include the step of extending sperm cells obtained from a male of a species of mammal in INRA96, and specifically with respect to sperm cells obtained from the male of an equine species of mammal INRA96 can significantly increase sperm cell motility.

### EXAMPLE 3.

Ejaculates from 8 stallions were extended to 25 x 10⁶ sperm/ml in KMT, and 40-ml aliquots were placed at 5, 10, 15, 20, and 25°C for 18h. Samples were then processed similarly to methods of Example 2. Motility was evaluated both with and without 2 mM caffeine as a stimulant Flow-cytometric evaluation of % dead was done using propidium iodide staining.

**Table 3. Percentage of motile sperm after 18 h at varying temperatures (°C) (non-stim./stim.).**

| Temperature | Post- Shipping | Pre-Stain | Post-Stain | Post-High Dilution | % Dead |
|---|---|---|---|---|---|
| 5 | 63^{ab}/63^{ab} | 56^{a}/56 | 49^{a}/49^{a} | 45a/50^{ab} | 28^{ab} |
| 10 | 62^{ab}/63^{a} | 58^{a}/56 | 48^{ab}/52^{a} | 45^{a}/50^{ab} | 26^{ab} |
| 15 | 65^{a}/64^{a} | 56^{a}/54 | 45^{ab}/51^{a} | 45^{a}/51^{a} | 23^{a} |
| 20 | 59^{bc}/62^{ab} | 54^{ab}/56 | 42^{b}/48^{ab} | 41^{a}/47^{ab} | 23^{a} |
| 25 | 56^{c}/59^{b} | 49^{b}/53 | 35^{c}/43^{b} | 35^{b}/44^{b} | 31^{b} |

| | | | | | |
|---|---|---|---|---|---|
| ^{a,b,c} Values in the same column without common superscripts differ (P<0.05). | | | | | |

As shown by Table 3, using KMT can reduce motility difference with respect to storage or transportation temperatures of 5, 10, or 15°C storage temperatures.

As such, the sperm cell process system invention can further include the step of diluting or maintaining such sperm cells obtained from the male of the species of mammal in such concentration(s) of KMT prior to staining such sperm cells with Hoechst stain and prior to flow sorting of such sperm cells

### EXAMPLE 4.

Sperm cells in ejaculates from three stallions were initially evaluated for volume, concentration, and motility. The remaining portion of the ejaculates were extended with either KMT or EZ mixin with either 0% additional seminal plasma or 10% seminal plasma by concentration to the following sperm cell/stain concentrations: 50 x10⁶ sperm/mL, 2.6µl Hoechst; 50 x10⁶ sperm/mL, 3.9µl Hoechst; 150 x10⁶ sperm/mL, 7.8µl Hoechst; or 450 x10⁶ sperm/mL, 23.4 µl Hoechst and processed either immediately or after 18 hours storage at room temperature. Stained sperm cell samples were then evaluated for resolution and % dead by flow cytometry analysis, and motility was evaluated by further diluting 20 µl of each stained sperm cell sample with 140 µl EZ Mixin or KMT.

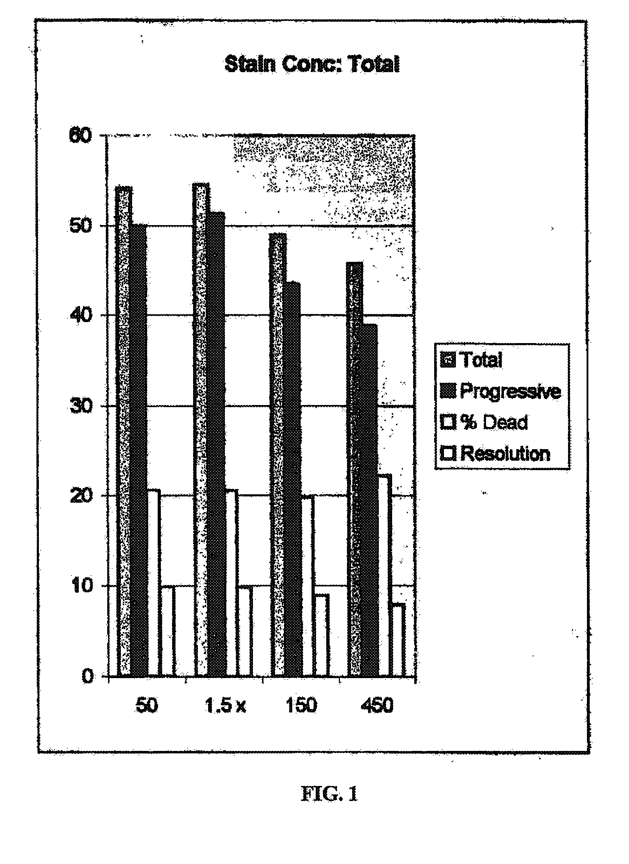

Now referring primarily to Figure 1, it can be understood that as stain concentration increases the total and progressive motility of sperm cells, and in particular equine sperm cells, decreases, % dead sperm cells increases, and the ability of flow cytometry techniques to resolve X-chromosome bearing sperm cells from Y-chromosome sperm cells increases.

The sperm cell process system invention can comprise a range of stain concentration(s) that provides enhanced total or progressive motility of stained sperm cells, resolution of X-chromosome bearing sperm cell from Y-chromosome bearing sperm cells during flow-sorting; or decrease in the % dead sperm cells, compared to the range of stain concentration used in conventional technology with respect to sperm cells obtained from a particular species, or other ranges of stain concentrations as disclosed. Specifically for equine applications of the invention, the range of stain concentration that can provide enhanced total or progressive, enhanced resolution of X-chromosome bearing sperm cell from Y-chromosome bearing sperm cells during flow-sorting; or a reduction of the % dead sperm cells in stained sperm cell samples can be between about 50 x10⁶ sperm/mL, 2.6µl Hoechst; 50 x10⁶ sperm/mL, 3.9µl Hoechst. While results are less favorable the range of stain concentration can be between 150 x10⁶ sperm/mL, 7.8µl Hoechst to about 450 x10⁶ sperm/mL, 23.4 µl Hoechst.

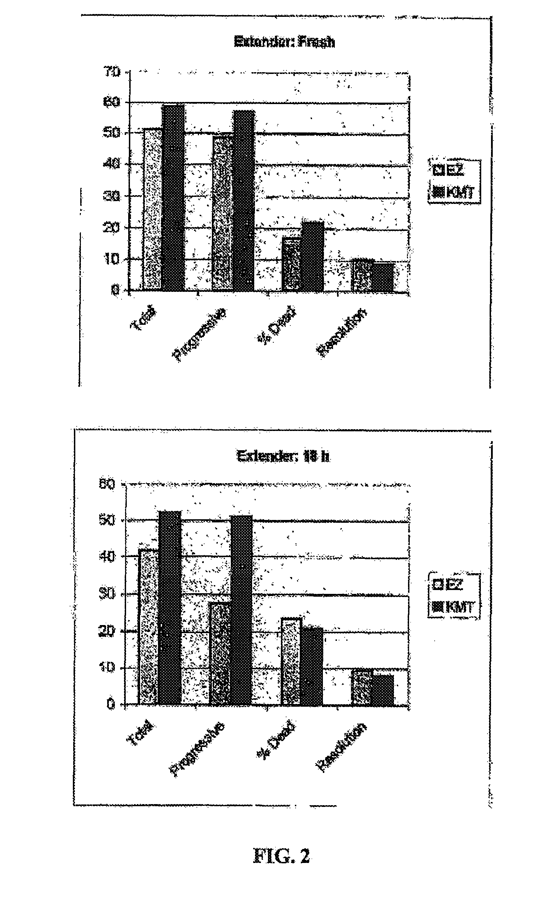

Now referring primarily to Figure 2, it can be understood that sperm cells extended in KMT remain more motile with respect to both fresh sperm samples and sperm samples stored for a duration of time at room temperature, such as 18 hours at room temperature.

The invention can further include use of KMT as an extender to increase total or progressive motility of fresh sperm cells, of sperm cells stored for a duration of time, for example up to 18 hours or longer, or of sperm cells that are transferred or transported from a first location, such as the location at which the sperm cells are obtained from a male mammal, to a second or a plurality of locations where further processing of sperm cells obtained from the male mammal occurs such as sperm cell counting, separation of X-chromosome bearing sperm cells from Y-chromosome bearing sperm cells, or preparation of sperm cell containing products including but not limited to the manufacture of straws of sperm for artificial insemination (whether sorted or not), or a second or plurality of locations where insemination of a female species of the mammal occurs, oocytes are fertilized in-vitro, or the like.

### EXAMPLE 5.

Sperm cells in ejaculates from three stallions were initially evaluated for volume, concentration, and motility. The remaining portion of each ejaculate was extended in KMT to 25 x10⁶ sperm/mL and stored at RT for 18 hr. The stored ejaculates were pelleted by centrifugation at 600g for 10 min. Pelleted sperm cells were resuspended in KMT with Hoechst to generate sperm samples of 400 x10⁶ sperm/mL,12.4 µl Hoechst, adjusted to either 7.1 pH or 7.9 pH, and then incubated at 34°C for either 30min or 60min. Stained sperm cell samples were extended with either 1mL KMT with 1.5 µl/mL 5% red food dye; 1mL KMT with 2.0 µl/mL 5% red food dye; 1mL KMT with 2.5 µl/mL 5% red food dye; or 1mL KMT with 3.0 µl/mL 2% red food dye. Sperm cell samples were then evaluated for % dead and resolution by flow cytometry analysis, and motility was evaluated by further diluting treated samples in either 140 µl KMT: 20 µl sperm cells; 140 µl KMT 2mM caffeine: 20 µl sperm cells; 140 µl KMT, 2.5 mM NaPyruvate: 20 µl sperm cells.

**Table 4. Effect of Stallion**

| | Gunsmoke | Rowdy | Sylekt |
|---|---|---|---|
| % Dead | 15.5 | 16 | 17.13 |
| Resolution | 5.81 | 4.5 | 5.88 |
| | | | |

| | Gunsmoke | Rowdy | Sylekt |
|---|---|---|---|
| Motility 0h | 55 | 68.44 | 59.69 |
| Progressive 0h | 43.13 | 68.44 | 56.25 |
| Motility 3h-None | 63.13 | 65.94 | 67.19 |
| Motility 3h-Caffeine | 66.25 | 67.5 | 69.38 |
| Motility 3h-Pyruvate | 62.19 | 67.19 | 65.94 |
| Progessive 3h-None | 55.94 | 65.94 | 63.75 |
| Progressive 3h-Caffeine | 63.13 | 67.5 | 69.06 |
| Progressive 3h-Pyruvate | 56.56 | 67.19 | 63.13 |

**Table 5. Effect of Stain pH**

| | | |
|---|---|---|
| | 7.1 | 7.9 |
| % Dead | 17.25 | 15.17 |
| Resolution | 5.58 | 5.21 |
| | | |
| | 7.1 | 7.9 |
| Motility 0h | 62.08 | 60 |
| Progressive 0h | 55.83 | 56.04 |
| Motility 3h-None | 66.25 | 64.58 |
| Motility 3h-Caffeine | 69.54 | 66.88 |
| Motilily 3h-Pyruvate | 65.83 | 64.38 |
| Progessive 3h-None | 62.29 | 61.46 |
| Progressive 3h-Caffeine | 67.5 | 65.63 |
| Progressive 3h-Pyruvate | 63.13 | 61.46 |

**Table 6. Effect of Food Coloring**

| | | | | |
|---|---|---|---|---|
| | 1.5 | 2 | 2.5 | 3 |
| % Dead | 16 | 16.33 | 16.17 | 16.33 |
| Resolution | 5.33 | 5.25 | 5.5 | 5.5 |
| | | | | |
| | 1.5 | 2 | 2.5 | 3 |
| Motility 0h | 60.42 | 62.08 | 62.5 | 59.17 |
| Progressive 0h | 55.83 | 56.67 | 57.08 | 54.17 |
| Motility 3h-None | 64.55 | 65.42 | 66.25 | 65.42 |
| Motility 3h-Caffeine | 66.67 | 68.33 | 68.75 | 67.08 |
| Motility 3h-Pyruvate | 64.58 | 65 | 65.83 | 65 |
| Progessive 3h-None | 61.67 | 61.67 | 62.92 | 61.25 |
| Progressive 3h-Pyruvate | 65.42 | 67.5 | 67.92 | 65.42 |
| Progressive 3h-Caffeine | 62.08 | 60.83 | 64.58 | 61.67 |

**Table 7. Effect of Stain Time (12 Samples only)**

| | 30min | 60 min |
|---|---|---|
| % Dead | 14.83 | 16.17 |
| Resolution | 4.92 | 5.5 |

| | 30 min | 60 min |
|---|---|---|
| Motility 0h | 66.25 | 62.5 |
| Progressive 0h | 62.08 | 57.08 |
| Motility 31h-None | 65.83 | 66.25 |
| Motility 3h-Caffeine | 66.67 | 68.75 |
| Motility 3h-Pyruvate | 64.58 | 65.83 |
| Progessive 3h-None | 62.08 | 62.92 |
| Progressive 3h-Caffeine | 65 | 67.92 |
| Progressive 3h-Pyruvate | 60.42 | 64.58 |

**Table 8. pH and Stain Time**

| | | | | |
|---|---|---|---|---|
| | 7.1 | | 7.9 | |

| | 30 min | 60 min | 30 min | 60 min |
|---|---|---|---|---|
| % Dead | 15.67 | 17 | 14 | 15.33 |
| Resolution | 4.83 | 5.83 | 5 | 5.17 |
| | | | | |
| | 7.1 | | 7.9 | |

| | 30 min | 60 min | 30 min | 60 min |
|---|---|---|---|---|
| Motility 0h | 65.83 | 62.5 | 66.67 | 62.5 |
| Progressive 0h | 61.67 | 55.83 | 62.5 | 58.33 |
| Motility 3h-None | 65.83 | 68.33 | 65.83 | 64.17 |
| Motility 3h-Caffeine | 66.67 | 70 | 66.67 | 67.5 |
| Motility 3h-Pyruvate | 64.17 | 67.5 | 65 | 64.17 |
| Progessive 3h-None | 63.33 | 64.17 | 60.83 | 61.67 |
| Progressive 3h-Caffeine | 65 | 70 | 65 | 65.83 |
| Progressive 3h-Pyruvate | 60.83 | 66 67 | 60 | 62.5 |

**Table 9. Motility Stimulants**

| | None | Caffeine | Pyruvate |
|---|---|---|---|
| Motility 3h | 65.42 | 67.71 | 65.1 |
| Progressive 3h | 61.88 | 66.56 | 62.29 |

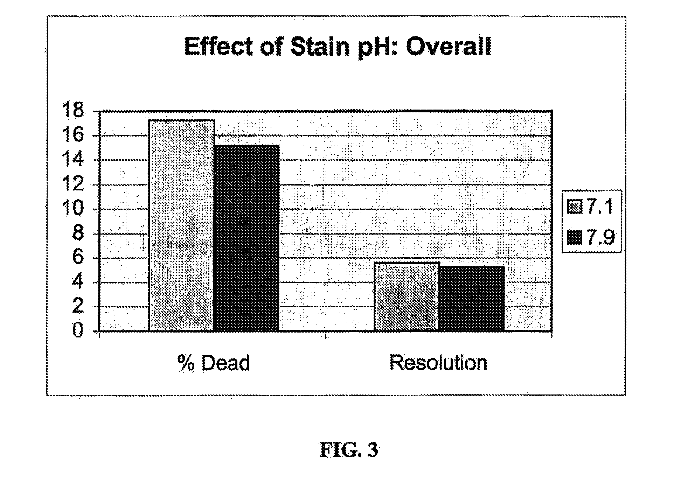

Now referring primarily to Figure 3, it can be understood that staining sperm cells at higher pH can decrease the percent dead sperm cells as evaluated by flow cytometry analysis.

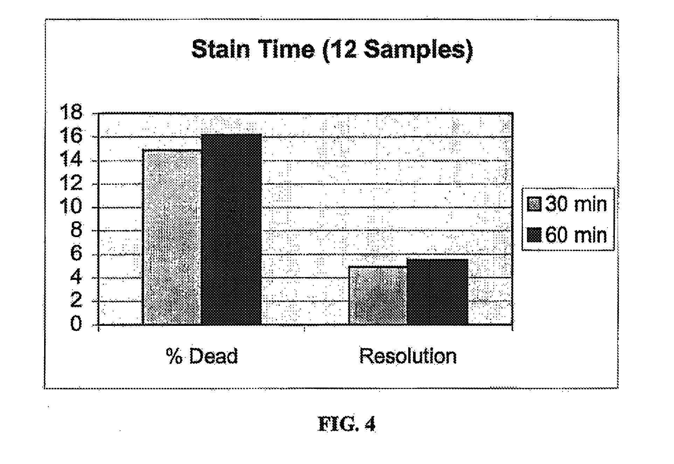

Now referring primarily to Figure 4, it can be understood that decreasing the incubation period to stain sperm cells from the conventional period of 60 minutes to a 30 minute period can decrease percent dead sperm cells, and increase resolution of X-chromosome bearing sperm cells from Y-chromosome bearing sperm cells during flow cytometry.

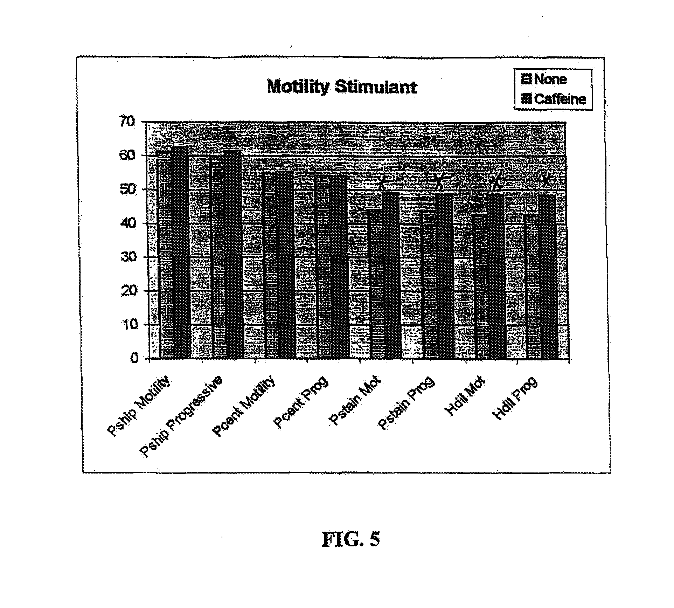

Now referring primarily to Figure 5 it can be understood that the addition of a stimulant such as caffeine at a concentration of about 2mM can stimulate motility in sperm cells and can be particularly effective in stimulating stressed equine sperm cells.

As such, the invention can further include the step of adjusting the pH of the solution in which sperm cells obtained from the male of a species of mammal are stained with a flourochrome, such as Hoechst. The pH of the stain solution can be raised to a pH between about 7.2 pH to about 8.0 pH to select the pH desired for a particular sperm cell sample or the one that generates a reduced or least % dead in a particular type of stained sperm cell sample. Specifically, with respect to sperm cells obtained from equine males the pH of the stain solution can be raised to between about 7.5 pH to about 8.0 pH and specifically can be 7.9 pH to reduce the % dead equine sperm cells in stained sperm cell samples.

The invention can further include the step of reducing the period of time in which sperm cells are incubated in the stain solution to reduce the % dead in stained sperm samples or to increase motility, or increase resolution of X-chromomsome bearing stained sperm from Y-chromosome bearing stained sperm when flow sorted or otherwise separated based on DNA content. Depending on the application in which the invention is employed, the period of time in which sperm cells are exposed to, incubated in, or are otherwise suspended in stain solution can be substantially reduced. The reduction in time can be 10%, 20%, 30%, 40%, 50%, or more from the amount of time typically used; or can be a reduction in time that reduces the number of dead sperm cells resulting from staining with a fluorochrome without a significant reduction in resolution during flow sorting; or can be a reduction in time that results in increased resolution during flow sorting without a significant increase in the % dead sperm cells in the stained sample. The amount of time that an equine sperm cell sample, for example, incubates in a staining solution of Hoechst (as described above) can be between about 25 minutes to about 50 minutes to obtain greater flow sorting resolution or reduced % dead sperm cells, and can specifically be 30 minutes. The actual reduction in time can be determined to provide a desired balance between motility, % dead sperm cells, and flow sort resolution within a population of sperm cells proximate to the time of stained.

The invention can further include the step of adding a stimulant to the sperm cell sample, The stimulant can be caffeine, or a stimulant similar to caffeine, or a stimulant that increases sperm cell motility or other sperm cell function or characteristic, whether mechanical or physiological. The stimulant can be added prior to or after the sperm cell is exposed to a process step such as storage, transportation, dilution, flow sorting, insemination, or the like. Specifically, a concentration of between about 1mM and about 5mM caffeine can be used and specifically with respect to equine sperm cells a 2mM concentration of caffeine can be used.

### EXAMPLE 6.

Ejaculates from ten stallions were initially evaluated for volume and sperm cell concentration and motility, The remaining portion of each ejaculate was extended in either KMT prepared using Na₂H₂PO₄ KMT or KMT prepared using NaH₂PO₄ (KMT-mod.) to 25 x10⁶ sperm/mL and stored at 5°C, 10°C, 15°C, 20°C, 25°C for 18 hr. A 100 µl aliquot of treated sperm cells were then diluted with 100 µl KMT or 100 µl KMT, 4mM caffeine and motility of the sperm cells was evaluated. The remaining portion of the treated sperm cells in each sample were centrifuged at 600g for 10 min. the supernatant aspirated to about 0.75 mL and the pelleted sperm cells resuspended in that volume. Post centrifugation motility of treated sperm cells was evaluated after dilution of 20 µl of each sample with either 520 µl KMT or 520 µl KMT, 2mM caffeine.

Aliquots of 200 x10⁶ sperm/mL, 12.4 µl Hoechst of each treatment group adjusted to 7.1 pH were incubated at 34°C for 60min. Stained sperm cell samples were extended with either 1mL, KMT with 1.5 µl/mL 5% red food dye. High dilution samples were then prepared by addition of 3mL KMT or 3mL KMT-mod. and 22mL 5mMHBGM-3 into 175 µl stained sperm (at 100 x10⁶ sperm/mL).

Treated sperm cell samples were then evaluated four % dead and resolution by flow cytometry analysis, and motility was evaluated by further diluting treating samples in either 140 µl KMT: 20 µl sperm cells; or 140 µl KMT 2nM caffeine: 20 µl sperm cells.

**Table 10. Effect of Stallion**

| | Pship | Pcent | Pstain | Hdil | %dead | resolution |
|---|---|---|---|---|---|---|
| A | 63.3 | 52.5 | 44.5 | 47.5 | 22.8 | 5.7 |
| B | 54.5 | 50.8 | 323 | 31.8 | 41.1 | 8.2 |
| C | 68.8 | 62.8 | 55.6 | 44.1 | 21.4 | 6.2 |
| D | 64.7 | 58.4 | 46.6 | 48.4 | 26.1 | 7.3 |
| E | 65.3 | 57.8 | 48.8 | 45.5 | 21.6 | 5.2 |
| G | 58.8 | 58 | 36.8 | 37 | 30.7 | 7 |
| H | 58 | 51.5 | 48.5 | 46.5 | 25.2 | 8 |
| J | 58 | 50 | 41.8 | 41.8 | 17.9 | 6.8 |

**Table 11. Effect of Extender**

| | Pship | Pcent | Pstain | Hdil | %Dead | Resolution |
|---|---|---|---|---|---|---|
| KMT | 60.7 | 52.8 | 42.8 | 40.9 | 26.6 | 6.8 |
| KMT Mod | 61.6 | 57.1 | 45.2 | 44.4 | 25.5 | 6.8 |

**Table 12. Effect of Shipping Temperature**

| | Pship | Pcent | Pstain | Hdil | %Dead | Resolution |
|---|---|---|---|---|---|---|
| 5 | 62.5 | 56.1 | 48.8 | 45 | 27.9 | 6.9 |
| 10 | 62 | 58 | 47.5 | 44.7 | 26.4 | 6.8 |
| 15 | 64.7 | 56.3 | 44.5 | 44.8 | 23.1 | 6.7 |
| 20 | 59.2 | 53.6 | 42.2 | 41.4 | 23.3 | 6.6 |
| 25 | 55.8 | 49.4 | 34.6 | 35.4 | 31.4 | 7.2 |

**Table 13. Post-Ship Motilily**

| | Total | Prog. | Stim., | T Stim.,Prog |
|---|---|---|---|---|
| 5 | 62.5 | 59.2 | 62.5 | 61.4 |
| 10 | 62 | 58.9 | 63.3 | 61.9 |
| 15 | 64.7 | 62.7 | 642 | 62.7 |
| 20 | 59.2 | 59.1 | 62.3 | 61.1 |
| 25 | 55.8 | 55 | 59 | 57.9 |

**Table 14. Post-Centrifugation Motility**

| | Total | Prog. | Stim. | T Stim. Prog |
|---|---|---|---|---|
| 5 | 56.1 | 55.6 | 56.3 | 55.2 |
| 10 | 58 | 56.3 | 56.4 | 54.4 |
| 15 | 56.3 | 56.1 | 53.6 | 52.2 |
| 20 | 53.6 | 52.8 | 56.4 | 55.6 |
| 25 | 49.4 | 48.5 | 52.7 | 51.9 |

**Table 15. Post-Staining Motility**

| | Total | Prog. | Stim., T Stim., | Prog |
|---|---|---|---|---|
| 5 | 48.8 | 48.6 | 49.1 | 49.1 |
| 10 | 47.5 | 47.2 | 52 | 51.9 |
| 15 | 44.5 | 44.5 | 51.4 | 51.3 |
| 20 | 42.2 | 42.2 | 48.1 | 47.5 |
| 25 | 34.6 | 34 | 42.7 | 42.7 |

**Table 16. Post-High Dilution Motility**

| | Total | Prog. | Stim., | T Stim., Prog |
|---|---|---|---|---|
| 5 | 45 | 45 | 49.5 | 49.5 |
| 10 | 44.7 | 44.1 | 49.5 | 48.9 |
| 15 | 44.8 | 44.8 | 51.1 | 51.1 |
| 20 | 41.4 | 40.5 | 46.9 | 46.9 |
| 25 | 35.4 | 36.9 | 44.4 | 43.3 |

**Table 17. Percent Dead and Resolution**

| | % Dead | Resolution |
|---|---|---|
| 5 | 27.9 | 6.9 |
| 10 | 26.4 | 6.8 |
| 15 | 23.1 | 6.7 |
| 20 | 23.3 | 6.6 |
| 25 | 31.4 | 7.2 |

**Table 18. KMT vs mod, Post-ship motility**

| | KMT | mod |
|---|---|---|
| 5 | 62.8 | 62.2 |
| 10 | 61.9 | 62.2 |
| 15 | 63.4 | 65.9 |
| 20 | 57.8 | 60.6 |
| 25 | 56.3 | 55.4 |

**Table 19. KMT vs mod, High Dilution motility**

| | KMT | mod |
|---|---|---|
| 5 | 42.8 | 47.2 |
| 10 | 44.4 | 45 |
| 15 | 43.1 | 46.6 |
| 20 | 40 | 42.8 |
| 25 | 31.7 | 39.2 |

**Table 20. KMT vs KW-mod, Percent dead**

| | KMT | mod |
|---|---|---|
| 5 | 27.5 | 28.4 |
| 10 | 26.4 | 26.5 |
| 15 | 23.3 | 23 |
| 20 | 23 | 23.6 |
| 25 | 36.8 | 26 |

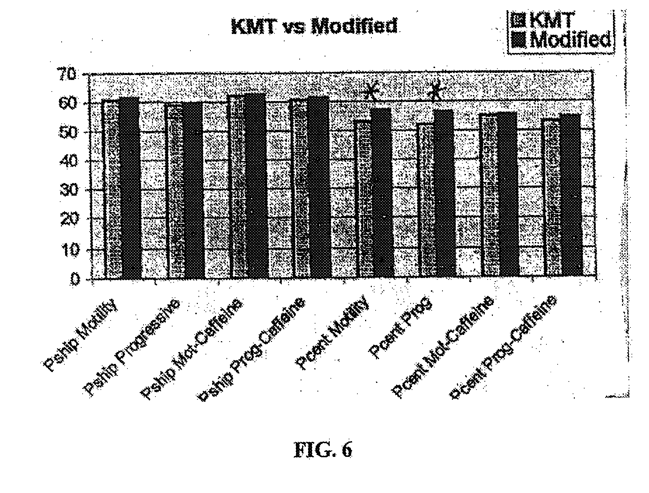

Now referring primarily to Figure 6, total motility and progressive motility of sperm cells shows results of using modified KMT prepared using NaH₂PO₄.

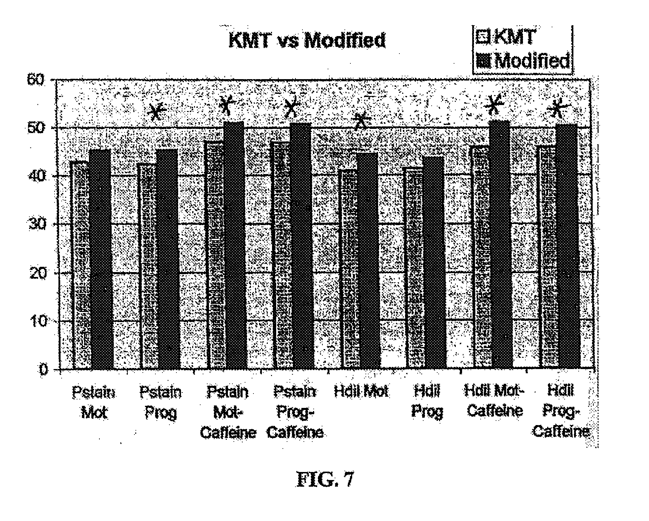

Now referring primarily to Figure 7 it can be understood that total motility and progressive motility of sperm cells after process steps such as staining for flow sorting and steps in which sperm cells are diluted may not be increased using modified KMT prepared using NaH₂PO₄ whether or not the sperm cells are exposed to stimulant such as caffeine.

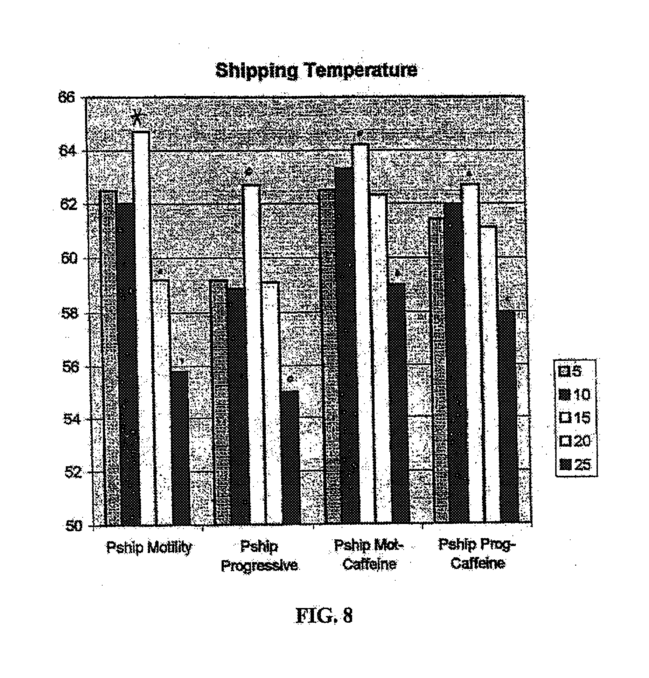

Now referring primarily to Figure 8 it can be understood that temperature can be adjusted for storing, handling, transferring, or transportation of sperm cells obtained from a male of a species of mammal to increase total and progressive motility. With respect to some sperm cells from certain species of mammals, storing, handling, transferring, or transportation at about 15°C can maintain highest levels of total or progressive motility of sperm cells or stimulated sperm cells.

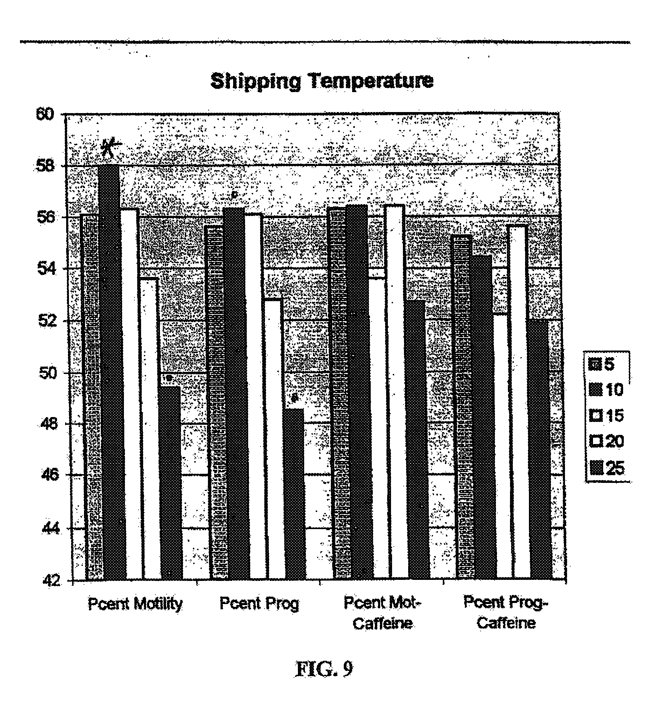

Now referring to Figure 9, it can be understood that the temperature at which spenn cells are transferred, stored, or handled prior to a staining protocol, such as described above, can be adjusted to increase total or progressive motility of sperm cells, or stimulated sperm cells, or sperm cells stimulated with caffeine. With respect to certain embodiments of the invention, including those in which equine sperm cells are processed, storage, transfer, or transport temperatures of between about 5°C to about 20°C can increase total and progressive motility of sperm cells. Moreover, with respect to stimulated sperm cells processed in accordance with the invention, including those embodiments of the invention in which equine sperm cells are stimulated with caffeine, handling, storage, or transfer temperatures between 5°C to about 20°C can also increase total and progressive motility. Specifically, embodiments of the invention used to process stimulated equine sperm cells comprises temperatures between about 10°C to about 15°C for handling, storing, or transferring of stimulated equine sperm cells.

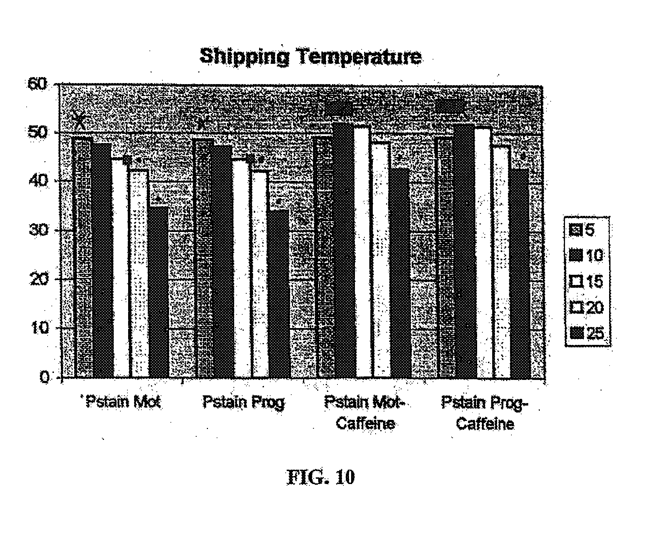

Now referring to Figure 10, it can be understood that the temperature at which sperm cells are transferred, stored, or handled prior to a staining protocol, such as described above, can be adjusted to increase total or progressive motility of sperm cells, or stimulated sperm cells, or sperm cells stimulated with caffeine subsequent to a staining protocol. With respect to certain embodiments of the invention, including those in which equine sperm cells are processed, storage, transfer, or transport temperatures of between about 5°C to about 20°C can increase total and progressive motility of sperm cells subsequent to staining protocols. Moreover, with respect to stimulated sperm cells processed in accordance with the invention, including those embodiments of the invention in which include equine sperm cells stimulated with caffeine, handling, storage, or transfer temperatures between 5°C to about 20°C can also increase total and progressive motility. Specifically, embodiments of the invention used to process stimulated equine sperm cells comprise temperatures between about 10°C to about 15°C for handling, storing, or transferring of stimulated equine sperm cells.

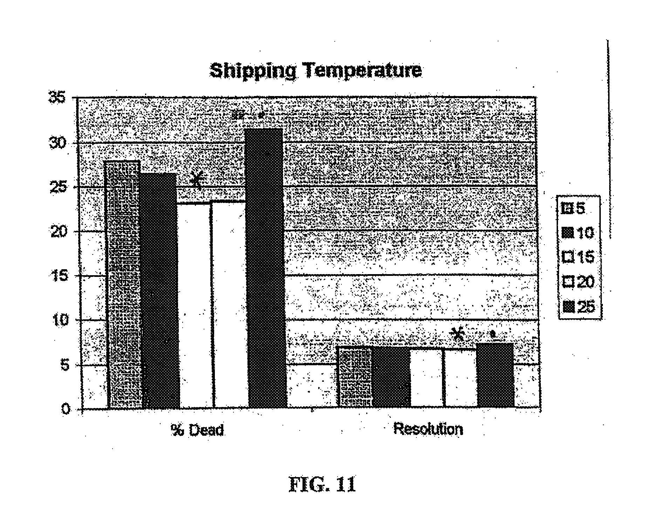

Now referring to Figure 11, it can be understood that % dead of sperm cells after staining as described above can be reduced by storing or transporting sperm cells at 15°C.

### EXAMPLE 7.

Ejaculates from twelve stallions were initially evaluated for volume and sperm cell concentration and motility. The remaining portion of each ejaculate was extended in KMT to 25 x10⁶ sperm/mL and stored at 15°C for 18 hr. Post storage motility was evaluated using 100 µl aliquots of treated sperm cells were then diluted with 100 µl KMT, 4mM caffeine. The remaining portion of the treated sperm cells in each sample were centrifuged at 600g for 10 min. the supernatant aspirated to about 1.50 mL and the pelleted sperm cells resuspended in that volume. Treated sperm cells were extended to 400 x10⁶ sperm/mL and aliquots transferred to a staining tube for treatment as follows:
1. 200 x10⁶ sperm/mL, 8.68 µl Hoechst 7.1 pH, incubated at 34°C for 60min
2. 200 x10⁶ sperm/mL, 10.54 µl Hoechst 7.1 pH, incubated at 34°C for 60min
3. 200 x10⁶ sperm/mL, 12.44 µl Hoechst 7.1 pH, incubated at 34°C for 60min
4. 200 x10⁶ sperm/mL, 8.68 µl Hoechst 7.1 pH, incubated at 34°C for 30min
5. 200 x10⁶ sperm/mL,10.54 µl Hoechst 7.1 pH, incubated at 34°C for 30min
6. 200 x10⁶ sperm/mL,12.44 µl Hoechst 7.1 pH, incubated at 34°C for 30min
7. 100 x10⁶ sperm/mL, 4.34µl Hoechst 7.1 pH, incubated at 34°C for 60min
8. 100 x10⁶ sperm/mL, 5.27 µl Hoechst 7.1 pH, incubated at 34°C for 60min
9. 100 x10⁶ sperm/mL, 6.22 µl Hoechst 7.1 pH, incubated at 34°C for 60min
10. 100 x10⁶ sperm/mL, 4.34 µl Hoechst 7.1 pH, incubated at 34°C for 30min
11. 100 x10⁶ sperm/mL, 5.27 µl Hoechst 7.1 pH, incubated at 34°C for 30min
12. 100 x10⁶ sperm/mL, 6.22 µl Hoechst 7.1 pH, incubated at 34°C for 30min

Each stained sperm sample was diluted to 75 x10⁶ sperm/mL with KMT, 0.75 µl/mL 5% red food dye. High dilution samples were then prepared by addition of 3mL KMT and 22mL 5mM HBGM-3 into 234µl stained sperm cell sample (at 75 x10⁶ sperm/mL). Each stained sperm cell sample was then evaluated for % dead and resolution by flow cytometry analysis, and motility was evaluated in KMT and KMT, 2mM caffeine.

High dilution samples were then prepared by addition of 3mL KMT and 22mL 5mM HBGM-3 into 234µl stained sperm cell sample (at 75 x10⁶ sperm/mL) and incubated at RT for about 1.5 hr. High dilution sperm cell samples were then evaluated for motility in KMT and in KMT, 4mM caffeine

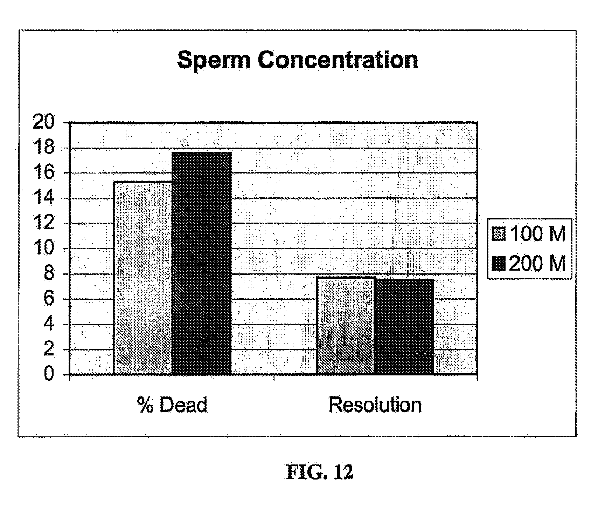

Now referring primarily to Figure 12, it can be understood that sperm cells remain more viable when sperm cell concentration during staining is at about 100 M/mL versus 200M/mL without loss of resolution.

Certain embodiments of the sperm cell process system invention can further include the step of diluting sperm cells obtained from a male of a species of mammal to between about 75 M/mL and 200 M/mL to obtain a concentration of sperm cells that reduces, minimize, or in which % dead in the sample after staining does not decrease with further increase in dilution of the sperm cells, Specifically, with respect to some embodiments of the invention the concentration of sperm cells can be less than 200M/mL and with respect to equine sperm cells can be about 100M/mL to reduce the number of % dead as evaluated by flow cytometry subsequent to the above described staining procedure.

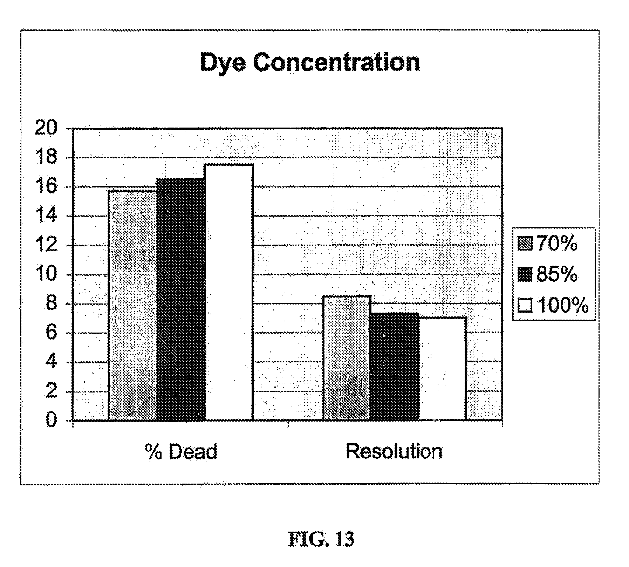

Now referring primarily to Figure 13, it can be understood that as stain concentration increases fewer sperm cells survive and resolution increases.

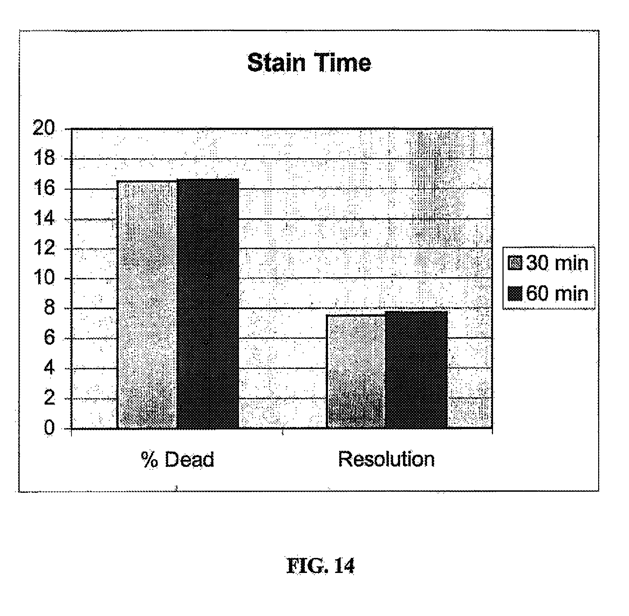

Now referring primarily to Figure 14, it can be understood that stain time can be substantially decreased without loss of resolution between X-chromosome bearing populations and Y-chromosome bearing populations of sperm cells evaluated by flow cytometry.

As such, embodiments of the invention can further include the step of decreasing the stain concentration used in the stain protocol described above until the % dead in the stained sperm cell samples does not substantially decrease further, and can further include the step of decreasing the stain concentration used until the resolution of X-chromosome bearing sperm cell from Y-chromosome bearing flow cells yields a sorted sperm cell sample of less than 60% purity; or less than the % purity necessary or desired, such as 60%, 65%, 70%, 75%, 80%, 85%, 90%, 91%, 92%, 93%,94%, 95%, 96%, 97%, or 98%; or less than that which can be achieved for sperm cells from that species of male mammal; or not less than flow sorting one of or both of X-chromosome bearing or the Y-chromosome bearing from one another at a sort rate of not less than between about 500 sorts/sec to about 1000 sorts/second, between about 750 sorts/sec to about 1250 sorts/second, between about 1000 sorts/sec to about 1500 sorts/sec; between about 1250 sorts/sec to about 1750 sorts/sec; between about 1500 sorts/sec to about 2000 sorts/sec; between about 1750 sorts/sec to about 2250 sorts/sec; between about 2000 sorts/sec to about 2500 sorts/sec; between about 2250 sorts/sec to about 2750 sorts/sec; between about 2500 sorts/sec to about 3000 sorts/sec; between about 2750 sorts/sec to about 3250 sorts/sec; between about 3000 sorts/sec to about 3500 sorts/sec; between about 3250 sorts/sec to about 3750 sorts/sec; between about 3500 sorts/sec to about 4000 sorts/sec; between about 3750 sorts/sec to about 4250 sorts/sec; between about 4000 sorts/sec to about 4500 sorts/sec; between about 4250 sorts/sec to about 4750 sorts/sec; between about 4500 sorts/sec to about 5000 sorts/sec.

### EXAMPLE 8. INSEMINATION OF COW ELK WITH SEXED FROZEN SEMEN

Cow elk 3-6-yr of age in Colorado and Minnesota were synchronized for estrus in September by insertion of a progesterone CIDR into the vagina for 12-14 d. Upon removal of the CIDR, 200 IU of eCG was administered intramuscular and elk were timed-inseminated 60 h later. Fresh semen was collected via electro-ejaculation from a 5-yr old bull elk and slowly cooled over 4 h to about 20°C for transportation as a neat ejaculate to the sperm-sorting laboratory. The ejaculate was concentrated to 1 x 10⁹ sperm/ml for straining by centrifuging 1.5 ml aliquots for 10 sec at 15,000 x g. Semen was incubated in 112 µM Hoechst 33342 at 200 x 10⁶ sperm/ml in a TALP medium for 45 min at 34°C, and then diluted to 100 x 10⁶/ml for sorting. Sperm were sorted on the basis of differing DNA content of X and Y chromosome-bearing sperm. X chromosome-bearing elk sperm contained 3.8% more DNA than Y chromosome-bearing sperm. Sperm were flow-sorted over a 4 h period using MoFlo®SX operating at 50 psi with a TRIS-based sheath fluid. The 351 and 364 bands of an argon laser, emitting 150 mW, excited Hoechst 33342 dye bound to DNA. Both X and Y chromosome-bearing sperm were collected (∼92% purity as verified by reanalyzing sonicated sperm aliquots for DNA) were collected at ∼4,700 sperm/sec into tubes containing 2 ml of 20% egg yolk-IRIS extender. Sorted volumes of 15 ml were sequentially collected. Approximately 110 x 10⁶ spenn of each sex were sorted and cooled to 5 °C over 90 min. An equal volume of glycerol (12%) containing extender was added to the sorted volume at 5°C, Sorted sperm aliquots containing 30-ml were concentrated by centrifugation at 4 °C for 20 min at 850 x g. Sperm pellets were pooled, adjusted to 21.7 x 10⁶ sperm/ml and loaded into 0.25-ml straws. Each straw, containing 5 x 10⁶ total sperm, was frozen in liquid nitrogen vapor. As a control. 5 x 10⁶ total sperm from the same ejaculate were frozen in 0.25 ml straws at the same time as the sexed sperm. After thawing for 30 sec at 37°C, 65% and 60% of sperm (control and sexed, respectively) were progressively motile as determine by visual estimates. Cows at 3 different locations and management schemes were inseminated using routine trans-cervical semen deposition in the uterine body. Pregnancy was determined 40-d post insemination by assaying blood for Pregnancy-Specific Protein B (Bio Tracking, Moscow, Idaho). Ten cows at one location were in poor condition at the time of insemination and no pregnancies were achieved with sexed or control sperm. The pregnancy rate at the other locations with sexed sperm (61%; 11/18) was similar to that for control inseminates (50%; 3/6). These pregnancy rates (sexed and controls) resulted from fewer sperm than are used in normal elk artificial insemination. Nine of eleven (82%) of sexed calves were of the predicted sex.

The invention can further include a mammal produced in accordance with any of the above described embodiments of the invention, or can include a mammal of predetermined sex in accordance with the various embodiments of the invention that provide sperm cell insemination samples having an enriched population of either X-chromosome bearing sperm cells or enriched population of Y-chromosome bearing sperm cells, or a mammal produced in accordance with any embodiment of the invention in which a sperm cell insemination sample containing a low number of sperm cells compared to the typical number used to inseminate that particular species of mammal is used, or elk progeny produced in accordance with the invention as describe above.

As can be easily understood from the foregoing, the basic concepts of the present invention may be embodied in a variety of ways. It involves both a sperm cell process system including both techniques as well as devices to accomplish sperm cell processing. In this application, various sperm cell processing techniques are disclosed as part of the results shown to be achieved by the various devices described and as steps which are inherent to utilization. They are simply the natural result of utilizing the devices as intended and described. In addition, while some devices are disclosed, it should be understood that these not only accomplish certain methods but also can be varied in a number of ways. Importantly as to all of the foregoing, all of these facets should be understood to be encompassed by this disclosure.

The discussion included in this nonprovisional application is intended to serve as a basic description. The reader should be aware that the specific discussion may not explicitly describe all embodiments possible; many alternatives are implicit. It also may not fully explain the generic nature of the invention and may not explicitly show how each feature or element can actually be representative of a broader function or of a great variety of alternative or equivalent elements. Again, these are implicitly included in this disclosure, Where the invention is described in device-oriented terminology, each element of the device implicitly performs a function. Apparatus claims may not only be included for the device described, but also method or process claims may be included to address the functions the invention and each element performs. Neither the description nor the terminology is intended to limit the scope of the claims

Further, each of the various elements of the invention and claims may also be achieved in a variety of manners. This disclosure should be understood to encompass each such variation, be it a variation of an embodiment of any apparatus embodiment, a method or process embodiment, or even merely a variation of any element of these. Particularly, it should be understood that as the disclosure relates to elements of the invention the words for each element may be expressed by equivalent apparatus terms or method terms -- even if only the function or result is the same. Such equivalent, broaden or even more generic terms should be considered to be encompassed in the description of each element or action. Such terms can be substituted where desired to make explicit the implicitly broad coverage to which this invention is entitled. As but one example, it should be understood that all actions may be expressed as a means for taking that action or as an element which causes that action. Similarly, each physical element disclosed should be understood to encompass a disclosure of the action which that physical element facilitates. Regarding this last aspect, as but one example, the disclosure of a "flow-sorter" should be understood to encompass disclosure of the act of "flow-sorting" -- whether explicitly discussed or not -- and, conversely, were there effectively disclosure of the act of "flow-sorting", such a disclosure should be understood to encompass disclosure of a "flow-sorter" and even a "means for flow-sorting" Such changes and alternative terms are to be understood to be explicitly included in the description.

Any acts of law, statutes, regulations, or rules mentioned in this application for patent; or patents, publications, or other references mentioned in this application for patent are hereby incorporated by reference. In addition, as to each term used it should be understood that unless its utilization in this application is inconsistent with such interpretation, common dictionary definitions should be understood as incorporated for each term and all definitions, alternative terms, and synonyms such as contained in the Random House Webster's Unabridged Dictionary, second edition are hereby incorporated by reference.

Thus, the applicant(s) should be understood to claim at least i) each of the sperm cell processing devices as herein disclosed and described, ii) the related methods disclosed and described, iii) similar, equivalent, and even implicit variations of each of these devices and methods, iv) those alternative designs which accomplish each of the functions shown as are disclosed and described, v) those alternative designs and methods which accomplish each of the functions shown as are implicit to accomplish that which is disclosed and described, vi) each feature, component, and step shown as separate and independent inventions, vii) the applications enhanced by the various systems or components disclosed, viii) the resulting products produced by such systems or components, and ix) methods and apparatuses substantially as described hereinbefore and with reference to any of the accompanying examples, x) the various combinations and permutations of each of the elements disclosed, and xi) each potentially dependent claim or concept as a dependency on each and every one of the independent claims or concepts presented. In this regard it should be understood that for practical reasons and so as to avoid adding potentially hundreds of claims, the applicant may eventually present claims with initial dependencies only. Support should be understood to exist to the degree required under new matter laws -- including but not limited to European Patent Convention Article 123(2) and United States Patent Law 35 USC 132 or other such laws-- to permit the addition of any of the various dependencies or other elements presented under one independent claim or concept as dependencies or elements under any other independent claim or concept. Further, if or when used, the use of the transitional phrase "comprising" is used to maintain the "open-end" claims herein, according to traditional claim interpretation. Thus, unless the context requires otherwise, it should be understood that the term "comprise" or variations such as "comprises" or "comprising", are intended to imply the inclusion of a stated element or step or group of elements or steps but not the exclusion of any other element or step or group of elements or steps. Such terms should be interpreted in their most expansive form so as to afford the applicant the broadest coverage legally permissible.

### SPECIFIC EMBODIMENTS

1. A method of separating sperm cells, comprising: a. obtaining semen from a male of a species of mammal which contains a plurality of sperm cells; b. incubating said semen at a temperature above which sperm cell membrane lipids transition from a liquid phase to gel phase; c. determining a sperm cell characteristic of a plurality of said sperm cells: d. separating said sperm cells based upon said sperm cell characteristic; and e. collecting separated sperm cells.
2. A method of separating sperm cells as described in embodiment 1, wherein said temperature above which sperm cell membrane lipids transition from a liquid phase to gel phase comprises the step of incubating said semen at a temperature above which sperm cell membrane lipids transition to a gel phrase.
3. A method of separating sperm cells as described in embodiment 1, wherein said temperature above which sperm cell membrane lipids transition from a liquid phase to gel phase comprises the step of incubating said semen at a temperature which maintains said sperm cell membrane lipids in said liquid phase.
4. A method of separating sperm cells as described in embodiment 1, wherein said step of incubating said semen at a temperature above which sperm cell membrane lipids transition from a liquid phase to gel phase temperature comprises incubating said semen at a temperature between about 5°C and about 25°C.
5. A method of separating sperm cells as described in embodiment 1, wherein said temperature is selected from the group consisting of about 5°C, about 6°C, about 7°C, about 8°C, about 9°C, about 10°C, about 11 °C, about 12°C, about 13°C, about 14°C, about 15°C, about 16°C, about 17°C, about 18°C, about 19°C, about 20°C, about 21°C, about 22°C, about 23°C, about 24°C, and about 25°C.
6. A method of separating sperm cells as described in embodiment 1, wherein said species of mammal is selected from the group consisting of a bovine species of mammal, an equine species of mammal, an ovine species of mammal, a swine species of mammal, a canine species of mammal, a feline species of mammal, a deer species of mammal, an elk species of mammal, and a marine species of mammal.
7. A method of separating sperm cells as described in embodiment 1, wherein said species of mammal comprises a bovine species and wherein said step of incubating said semen at a temperature above which sperm cell membrane lipids transition from a liquid phase to gel phase temperature comprises incubating said semen at a temperature between about 17°C. and about 19°C.
8. A method of separating sperm cells as described in embodiment 1, wherein said species of mammal comprises a bovine species and wherein said step of incubating said semen at a temperature above which sperm cell membrane lipids transition from a liquid phase to gel phase temperature comprises incubating said semen at a temperature of about 17°C
9. A method of separating sperm cells as described in embodiment 1, wherein said species of mammal comprises an equine species and wherein said step of incubating said semen at a temperature above which sperm cell membrane lipids transition from a liquid phase to gel phase temperature comprises incubating said semen at a temperature of about 15°C.
10. A method of separating sperm cells as described in embodiment 1, wherein said step of incubating said semen at a temperature above which sperm cell membrane lipids transition from liquid phase to gel phase comprises incubating said semen at said temperature above which sperm cell membrane lipids transition from liquid phase to gel phase between about one hour to about 18 hours.
11. A method of separating sperm cells as described in embodiment 1, further comprising the step of transporting said semen from a first location to a second location during said step of incubating said semen at a temperature above which sperm cell membrane lipids transition from a liquid phase to gel phase.
12. A method of separating sperm cells as described in embodiment 1, further comprising the step of adding an antibacterial to said semen prior to said step of incubating said semen at a temperature above which sperm cell membrane lipids transition from a liquid phase to gel phase.
13. A method of separating sperm cells as described in embodiment 1, wherein said step of determining a sperm cell characteristic of a plurality of sperm cells within said semen comprises determining a sex characteristic of said sperm cells.
14. A method of separating sperm cells as described in embodiment 1, wherein said step of separating said sperm cells based upon said sperm cell characteristic comprises separating said sperm cells based upon said sex characteristic.
15. A method of separating sperm cells as described in embodiment 1, further comprising the step of extending semen with an extender selected from the group consisting of KMT, and INRA96.
16. A method of separating sperm cells as described in embodiment 1, further comprising the step of concentrating said sperm cells by removing a portion of seminal plasma
17. A method of separating sperm cells as described in embodiment 1, further comprising the step of staining said sperm cells
18. A method of separating sperm cells as described in embodiment 17, wherein said step of staining said sperm cells comprises staining DNA contained within said sperm cells.
19. A method of separating sperm cells as described in embodiment 18, wherein said step of staining DNA contained within said sperm cells comprises staining said DNA within said sperm cells with Hoechst 33342 stain.
20. A method of separating sperm cells as described in embodiment 19, wherein said step of staining said DNA within said sperm cells with Hoechst 33342 stain comprises incubating said sperm cells with Hoechst 33342 for a period of between about 30 minutes and about 1 hour.
21. A method of separating sperm cells as described in embodiment 1, wherein said step of separating said sperm cells based upon said sperm cell characteristic comprises separating said sperm cells using an instrument selected from the group consisting of a flow cytometer, and a cell sorter.
22. An artificial insemination sample, comprising: a. an artificial insemination device; b. a sperm cell extender; and c. semen obtained from a male of a species of mammal containing a plurality of sperm cells incubated at a temperature above which sperm cell membrane lipids transition from a liquid phase to a gel phase for a period of between one hour and 18 hours, and wherein said plurality of sperm cells are separated on the basis of a sex characteristic, and wherein a portion of said separated sperm cells are established in said sperm cell extender, and wherein said portion of said separated sperm cells established in said sperm cell extender are contained within said artificial insemination device.
23. An artificial insemination sample as described in embodiment 22, wherein said sperm cell extender comprises an egg yolk extender.
24. An artificial insemination sample as described in embodiment 22, wherein said sperm cell extender comprises an, egg yolk-TRIS extender.
25. An artificial insemination sample as described in embodiment 22, wherein said sperm cell extender comprises substantially equal volumes of said egg yolk-TRIS extender and egg yolk-TRIS extender/12% glycerol.
26. An artificial insemination sample as described in embodiments 23, 24, or 25, wherein said sperm cell extender has a volume of between about 0.1 millilitre and about 2.0 millilitre.
27. An artificial insemination sample as described in embodiment 22. wherein said artificial insemination device comprises an artificial insemination straw.
28. An artificial insemination sample as described in embodiment 22, wherein said portion of said separated sperm cells established in said sperm cell extender comprise between about one hundred thousand separated sperm cells and about 25 million separated sperm cells.
29. An artificial insemination sample as described in embodiment 28, wherein said portion of said separated sperm cells established in said sperm cell extender contained within said artificial insemination device is frozen.
30. An artificial insemination sample as described in embodiment 29, wherein said portion of said separated sperm cells frozen in said sperm cell extender contained within said artificial insemination device are thawed.
31. A method of producing an artificial insemination sample comprising the steps of. a. obtaining semen from a male of a species of mammal which contains a plurality of sperm cells; b. incubating said semen at a temperature above which sperm cell membrane lipids transition from a liquid phase to a gel phase; c. determining a sex characteristic of said plurality of sperm cells; d. separating said plurality of sperm cells based upon said sex characteristic; and e. establishing an artificial insemination sample containing separated sperm cells capable of fertilizing at least one egg within a female of said species of said mammal.
32. A method of fertilizing a mammal comprising the steps of: a. obtaining semen from a male of a species of mammal which contains a plurality of sperm cells; b. incubating said semen at a temperature above which sperm cell membrane lipids transition to a gel phase; c. determining a sex characteristic of said plurality of sperm cells; d. separating said plurality of sperm cells based upon said sex characteristic; e. establishing an artificial insemination sample containing separated sperm cells capable of fertilizing at least one egg within a female of said species of said mammal; f. inserting a portion of said insemination sample into a female of said species of said mammal; and g. fertilizing at least one egg within said female of said species of said mammal.
33. A method of producing an offspring mammal comprising the steps of: a. obtaining semen from a male of a species of mammal which contains a plurality of sperm cells; b. incubating said semen at a temperature above which sperm cell membrane lipids transition to a gel phase; c. determining a sex characteristic of said plurality of sperm cells; d. separating said plurality of sperm cells based upon said sex characteristic; e. establishing an artificial insemination sample containing separated sperm cells capable of fertilizing at least one egg within a female of said species of said mammal; f. inserting a portion of said insemination sample into a female of said species of said mammal: g. fertilizing at least one egg within said female of said species of said mammal; and h. producing an offspring mammal.

## Claims

1. A method of separating sperm cells including:
a) obtaining semen from a male of a species of a non-human mammal which contains a plurality of sperm cells;
b) incubating the semen at a temperature of between about 5°C and about 20°C for between about one hour to about 18 hours;
c) determining a sperm cell characteristic of a plurality of the sperm cells;
d) separating the sperm cells based upon the sperm cell characteristic; and
e) collecting separated sperm cells.

2. A method of separating sperm cells as claimed in claim 1, **characterised in that** the incubating temperature is between about 10°C and about 20°C.

3. A method of separating sperm cells as claimed in claim 1 or claim 2, **characterised in that** the semen is from an equine species of mammal.

4. A method of separating sperm cells as claimed in claim 1, claim 2, or claim 3, **characterised in that** the temperature is selected from the appropriate group consisting of about 5°C, about 6°C, about 7°C, about 8°C, about 9°C, about 10°C, about 11°C, about 12°C, about 13°C, about 14°C, about 15°C, abut 16°C, about 17°C, about 18°C, about 19°C and about 20°C

5. A method of separating sperm cells as claimed in claim 1, claim 2 or claim 4, **characterised in that** the species of mammal is selected from the group consisting of a bovine species of mammal, an equine species of mammal, an ovine species of mammal, a swine species of mammal, a canine species of mammal, a feline species of mammal a deer species of mammal an elk species of mammal and a marine species of mammal.

6. A method of separating sperm as claimed in any preceding claim, **characterised in that** the method further includes the step of adding an antibacterial to the semen prior to the step of incubating the semen.

7. A method of separating sperm as claimed in any preceding claim, **characterised in that** the step of determining a sperm cell characteristic of a plurality of sperm cells within the semen includes determining a sex characteristic of the sperm cells.

8. A method of separating sperm as claimed in any preceding claim, **characterised in that** the method further includes the step of extending semen with an extender selected from the group consisting of KMT, KMT supplemented with red food dye, KMT-mod, INRA96, TALP medium, Tyrode's-based skim milk-glucose extender, EZ Mixin CST extender, EZ Mixin supplemented with seminal plasma, Next Generation, egg yolk-TRIS extender, egg yolk-TRIS extender supplemented with glycerol, and HBGM-3.

9. A method of separating sperm as claimed in any preceding claim, **characterised in that** the method further includes the step of staining DNA contained within the sperm cells with Hoechst 33342 staining solution and subsequently incubating the stained sperm cells for a period of between about 25 min and about 50 min, or between about 25 min and about 60 min.

10. A method of separating sperm as claimed in claim 9, **characterised in that** the method further includes the step of adjusting the pH of the staining solution, the pH of the staining solution being about pH 7.1, between about 7.2 to 8.0, between about 7.5 to 8.0, and/or the value of 7.9.

11. A method of separating sperm as claimed in any preceding claim, **characterised in that** the method further includes the step of adding caffeine, a stimulant similar to caffeine, pyruvate, or NaPyruvate.

12. A method of separating sperm as claimed in any preceding claim, **characterised in that** the method further includes one or more of the steps of:
a) establishing an artificial insemination sample containing separated sperm cells capable of fertilising at least one egg within a female of the species of the said mammal; and
b) inserting a portion of the insemination sample into a female of the species of the mammal.

13. The use of a sex-selected non human mammalian sperm artificial insemination sample for the manufacture of a formulation for artificial insemination **characterised in that** the sperm sample includes a sperm cell extender, the sperm being obtained from a male of a species of a non-human mammal containing a plurality of sperm cells incubated at a temperature of between about 5°C and about 20°C for between about one hour to about 18 hours and further **characterised in that** the plurality of sperm cells are separated on the basis of a sex characteristic and yet further **characterised in that** a portion of the separated sperm cells are established in the sperm cell extender.

14. The use of a sex-selected non-human mammalian sperm artificial insemination sample as claimed in claim 13, **characterised in that** the sperm cell extender is an egg yolk extender **characterised in that** the egg yolk extender includes an egg yolk-TRIS extender.

15. The use of a sex-selected non-human mammalian sperm artificial insemination sample as claimed in claim 14, **characterised in that** the egg yolk extender includes substantially equal volumes of the egg yolk-TRIS extender and an egg yolk-TRIS extender supplemented with glycerol.

16. The use of a sex-selected non-human mammalian sperm artificial insemination sample as claimed in any one of claims 13 to 15, **characterised in that** the portion of the separated sperm cells established in the sperm cell extender contained within the artificial insemination sample is frozen or frozen and subsequently thawed.

17. A method of separating sperm as in claim 1, **characterised in that** the sperm is taken from an equine, the pre-processing temperature is between about 10-15°C, the amount of stain used is in the range of between 2.6µl to 23.4µl stain per mL sperm cell sample; the staining pH is in the range of about pH 7.9 to about pH 8.0, the concentration of sperm during the staining process is less than about 200 M/mL, the staining period is between 25 and 50 minutes, and the extender is either KMT, KMT-mod or INTRA96 supplemented with caffeine, a caffeine-like stimulant, or a pyruvate stimulant.

18. A method of producing an embryo having a selected characteristic including the steps of:
a) providing a separated sperm cell sample according to any of the preceding claims;
b) combining the separated sperm cell sample with one or more oocytes of the same species either by artificial insemination or by fertilization in-vitro.
